# EUROPEAN PATENT APPLICATION

(11) **EP 0 573 087 A2**
(43) Date of publication of application: **08.12.1993**
(21) Application number: 93201285.9
(22) Date of filing: 06.05.1993
(51) Int. Cl.: C07C 45/41, B01J 23/34, B01J 37/03

(54) **Hydrogenation of benzoic acid and catalyst suitable therefor**

(30) Priority: 07.05.1992 NL 9200817
(71) Applicant: DSM N.V., NL-6411 TE Heerlen (NL)
(72) Inventor: van Geem, Paul Christiaan, NL-6174 RC Schinnen (NL); Xu, Xiaoding, NL-2623 MD Delft (NL); Scholten, Joseph Johannes Franciscus, NL-6132 BC Sittard (NL)

(57) **Abstract**

The invention relates to a catalyst that is very suitable for the conversion of a benzoic acid into the corresponding benzaldehyde. The catalyst can be obtained via coprecipitation of a manganese salt, a salt from which an acid support is formed, a zinc salt and optionally a copper salt at a pH between 4 and 10, through calcination, after precipitation, at a temperature of between 300 and 700°C and then, optionally, reduction with the aid of a hydrogen-containing gas mixture.

Using such a catalyst the hydrogenation can be carried out at lower temperatures resulting in energy-savings and, hence, cost-savings.

## Description

The invention relates to a catalyst suitable for the conversion of a benzoic acid into the corresponding benzaldehyde obtainable through coprecipitation of a manganese salt, a salt from which an acid support is formed, a zinc salt and optionally a copper salt at a pH between 4 and 10, followed by calcination of the precipitate at a temperature of between 300°C and 700°C and then, optionally, reduction with the aid of a hydrogen-containing gas mixture and to a process for the hydrogenation of benzoic acid using the same.

The hydrogenation in the presence of a catalyst containing manganese on an acid support is described in EP-A-290096. This catalyst is prepared via coprecipitation of a manganese salt and a salt from which an acid support is formed. A hydrogenation according to EP-A-290096 enables the production of a very high yield of benzaldehyde in the gas phase.

From an energy-saving and hence also cost-saving viewpoint it is desirable to carry out the hydrogenation at lower temperatures.

Zinc appears to be a suitable promoter to effect this. The presence of copper moreover enhances the effects of zinc. The selectivity and activity with respect to the benzaldehyde increase at lower reaction temperatures, which is important in connection with the prevention of the undesired formation of byproducts such as toluene, benzene and benzyl alcohol. If zinc and/or copper are used as promoters the hydrogenation reaction is mostly carried out at a temperature between 275 and 450°C, preferably between 300 and 400°C. The reaction is mostly carried out at atmospheric pressure or at a slightly elevated pressure e.g. up to 10 bar.
The catalyst used is an oxide of manganese zinc and optionally copper on an acid support in the form of an oxide.

As salt from which an acid support is formed use can be made of a salt from which on coprecipitation for example oxides of aluminium, zirconium, titanium, cerium, hafnium and/or niobium preferably of aluminium are formed. Preferably a nitrate of such an element is used. A suitable coprecipitator is for example NH₄OH or K₂CO₃.

The catalysts usually contain 2-40 wt.% manganese, calculated as metal with respect to the total amount of catalyst. The catalyst preferably contains 10-30 wt.% manganese. The catalysts moreover usually contain more than 10 wt.% of the metal, the oxide of which forms the acid support, e.g. Al, calculated as metal with respect to the total amount of catalyst, preferably 20-40 wt.%, and more than 1 wt.% Zn, calculated as metal with respect to the total amount of catalyst, preferably 15-50 wt.%, particularly 20-45 wt.%. The amount of Cu calculated as metal with respect to the total amount of catalyst mostly is lower than 5 wt.%, preferably 0,5-2 wt.%.

The process for the preparation of the catalyst using zinc and optionally copper does not differ substantially from the process without these promoters as described in EP-A-290096. The catalyst suitable for the conversion of a benzoic acid into the corresponding benzaldehyde is obtainable through coprecipitation of a manganese salt, a salt e.g. Al(N0₃)₃-from which an acid support e.g. Al₂O₃ is formed, a zinc salt and optionally a copper salt at a pH between 4 and 10, preferably 5-8, followed by precipitation and calcination at a temperature of between 300°C and 700°C and then optionally reduction with the aid of a hydrogen-containing gas mixture. The reduction with the aid of a hydrogen-contining gas mixture may also be carried out in situ under the hydrogenation reaction conditions. The soluble salts are mostly added to a solution of manganese salt, after which metal hydroxides are coprecipitated.

Suitable mangenese compounds are in particular soluble manganese salts such as manganese chloride, manganese bromide, manganese (II) sulphate, preferably manganese nitrate.

Suitable zinc compounds are in particular soluble zinc salts such as zinc nitrate, zinc sulphate, zinc chloride, zinc acetate.

Suitable copper compounds are in particular soluble copper salts such as copper nitrate, copper sulphate, copper chloride.

Such manganese oxide catalysts modified with promoters are suitable for the preparation of all kinds of benzaldehydes through hydrogenation of the corresponding benzoic acid. Benzoic acid may for example be substituted in one or more places in the aromatic ring with an alkyl group containing 1-6 C atoms, an alkoxy group containing 1-6 C atoms, a hydroxyl group and a halogen atom. Other substituents are also possible. The substituents may be at the ortho, meta and para positions. Examples of suitable benzoic acid compounds are o-Cl-benzoic acid, m-Cl-benzoic acid and p-t.butyl-benzoic acid. In the application 'benzoic acid' will be understood to include substituted benzoic acid too.

The hydrogenation of benzoic acid can be carried out in a manner known per se, under the influence of a hydrogenation catalyst and a gas mixture containing hydrogen. It can be carried out both batch wise and continuously.

After the hydrogenation of the benzoic acid the gas mixture may be cooled in order to condense the reaction products and excess hydrogen is recirculated. The water and benzene formed in the hydrogenation may be removed in an azeotropic drying column, after which benzaldehyde may be distilled.

The invention is explained with reference to the following, non-limiting, examples.

### Examples

### Preparation of the catalyst

Catalysts were prepared by dissolving Mn(NO₃)₂.4H₂O, Al(NO₃)₃.9H₂O, Cu(NO₃)₂.5H₂O and Zn(NO₃)₂.6H₂O in water to obtain a 1 M solution (relative to nitrate). Then a precipitate was formed by adding a 1 M solution of K₂CO₃ or NH₄OH to the solution under the conditions indicated in Table 1.

After filtration and drying the catalyst was reduced and screened to obtain 1-3-mm large particles. Then the catalyst was calcined at 200°C for 2 hours, followed by calcination at 500°C for 3 hours.

The compositions of these catalysts are as indicated in Table 1; Table 2 shows the results of a metal element analysis in wt%.

**TABLE 1**

| No. | catalyst preparation | | | composition |
|---|---|---|---|---|
| | solution pH | temp. | base | |
| 1 | 7.0 | 45 | K₂CO₃ | MnO₂/0.1 CuO 1.1 ZnO.ZnAl₂O₄ |
| 2 | 7.0 | 45 | K₂CO₃ | MnO₂/ZnAl₂O₄ |
| 3 | 7.0 | 45 | K₂CO₃ | MnO₂/ZnO ZnAl₂O₄ |
| 4 | 5.0 | 80 | K₂CO₃ | MnO₂/ZnO ZnA1₂O₄ |
| 5 | 8.0 | 45 | NH₄OH | MnO₂/ZnO ZnAl₂O₄ |
| 6 | 7.0 | 45 | NH₄OH | MnO₂/Al₂O₃ |

**TABLE 2**

| Cat. No. | Mn | Zn | Al | Cu |
|---|---|---|---|---|
| 1 | 10.6 | 27.0 | 22.5 | 1.2 |
| 2 | 11.2 | 26.6 | 23.1 | - |
| 3 | 11.2 | 36.0 | 15.8 | - |
| 4 | 3.5 | 42.5 | 19.0 | - |
| 5 | 13.0 | 31.0 | 19.8 | - |
| 6 | 10.1 | - | 36.1 | - |

### Examples I-XI

The benzoic acid was introduced into a saturator that was maintained at a temperature of 150°C. Hydrogen and nitrogen were added to the saturator containing benzoic acid.
The reactor with a diameter of 8 mm was provided with 5 ml of catalyst having a particle size of between 0.5 and 0.8 mm. The reactor was heated to a temperature of between 300 and 450°C with the aid of a tube furnace. The feed rate of the hydrogen was 35 Nl/h and that of the nitrogen was 4.75 Nl/h. The actual contact time of the reaction mixture was 0.2 seconds at 350°C. After the reactor the product stream (to which a DMF flow of 6.9 g/h had been added after the reaction) was collected in two coolers of 15 and -3°C for 30 minutes. Then the components were analysed.

Table 3 shows the temperature at which the reaction was carried out and the results that were obtained with catalysts 1-6.

**TABLE 3**

| Ex. | Cat. | Temp. (°C) | selectivity towards benzaldehyde | degree of conversion of benzoic acid |
|---|---|---|---|---|
| I | 1 | 330 | 88.3 | 98.9 |
| II | 2 | 350 | 95.2 | 85.7 |
| III | 2 | 380 | 78.2 | 100 |
| IV | 3 | 350 | 91.0 | 48.4 |
| V | 3 | 380 | 83.8 | 100 |
| VI | 4 | 350 | 95.0 | 53.2 |
| VII | 4 | 380 | 74.3 | 100 |
| VIII | 5 | 350 | 96.4 | 70.0 |
| IX | 5 | 380 | 79.7 | 100 |
| X | 6 | 414 | 84 | 89 |
| XI | 6 | 435 | 81 | 100 |

## Claims

1. Catalyst suitable for the conversion of a benzoic acid into the corresponding benzaldehyde obtainable through coprecipitation of a manganese salt, a salt from which an acid support is formed, a zinc salt and optionally a copper salt at a pH between 4 and 10, followed by calcination of the precipitate at a temperature of between 300°C and 700°C and then, optionally, reduction with the aid of a hydrogen-containing gas mixture.

2. Catalyst according to claim 1 wherein the amount of Mn calculated as metal with respect to the total amount of catalyst is 10-30 wt.%.

3. Catalyst according to claim 1 or 2, wherein the amount of Al calculated as metal with respect to the total amount of catalyst is 20-40 wt.%.

4. Catalyst according to any one of claims 1-3, wherein the amount of Zn calculated as metal with respect to the total amount of catalyst is 20-45 wt.%.

5. Catalyst according to any one of claims 1-4, wherein the amount of Cu calculated as metal with respect to the total amount of catalyst is 0,5-2 wt.%.

6. Process for the hydrogenation of benzoic acid by hydrogenating unsubstituted benzoic acid or a benzoic acid that is substituted in one or several places in the aromatic ring with an alkyl group containing 1-6 C atoms, an alkoxy group containing 1-6 C atoms, a hydroxyl group or a halogen atom, in the gas phase, with the aid of a catalyst according to any one of claims 1-5.

7. Use of a catalyst according to any one of calims 1-5 for the hydrogenation of benzoic acid or derivatives thereof.
